Europäisches Patentamt

**European Patent Office**

**Office européen des brevets**

(11) Numéro de publication: **0 183 603 B1**

## FASCICULE DE BREVET EUROPEEN

(45) Date de publication de fascicule du brevet: **19.08.92**

(51) Int. Cl.⁵: **C07C 29/70**, C07C 31/28, C07F 5/00

(21) Numéro de dépôt: **85402203.5**

(22) Date de dépôt: **14.11.85**

Le dossier contient des informations techniques présentées postérieurement au dépôt de la demande et ne figurant pas dans le présent fascicule.

(54) **Procédé de préparation d'alcoolates cériques.**

(30) Priorité: **26.11.84 US 674640**

(43) Date de publication de la demande: **04.06.86 Bulletin 86/23**

(45) Mention de la délivrance du brevet: **19.08.92 Bulletin 92/34**

(84) Etats contractants désignés: **AT BE CH DE FR GB IT LI NL SE**

(56) Documents cités: **EP-A- 0 134 161**

(73) Titulaire: **RHONE-POULENC CHIMIE 25, quai Paul Doumer F-92408 Courbevoie Cédex(FR)**

(72) Inventeur: **Gradeff, Peter P.O. Box 278 Pottersville New Jersey 08979(US)** Inventeur: **Schreiber, Fred 100 Lawrence Avenue Highland Park New Jersey 08904(US)**

(74) Mandataire: **Dutruc-Rosset, Marie-Claude et al RHONE-POULENC INTERSERVICES Service Brevets Chimie 25, Ouai Paul Doumer F-92408 Courbevoie Cédex(FR)**

## Description

La présente invention a pour objet un nouveau procédé de préparation d'alcoolstes cériques.

Les alcoolates de métaux polyvalents constituent une classe importante de composés organométalliques à multiples fonctions qui sont très utilisés dans l'industrie. Dans certains cas, leurs utilisations sont parallèles à celles des carboxylates de métaux et d'autres composés organométalliques, mais, par rapport à ces composés, ils présentent des avantages en raison de leurs propriétés catalytiques, leur facilité d'hydrolyse, leur solubilité dans les solvants organiques et leur volatilité. Ils ont été utilisés comme additifs pour peintures, agents hydrofuges, promoteurs d'adhérence, mordants, agents liants dans les compositions d'émaillage, catalyseurs et, ce qui est aussi très important, comme intermédiaires dans la synthèse d'autres composés organiques.

Il existe quatre méthodes générales de préparation des alcoolates métalliques, toutes mises en oeuvre dans des conditions anhydres, à savoir :

A. Réaction de l'alcool correspondant avec le métal tel que métaux alcalins, métaux alcalino-terreux et aluminium en présence d'un catalyseur alcalin ou acide.

B. Réaction de l'alcool correspondant avec les oxydes et hydroxydes métalliques tels que par exemple NaOH ou $Na_2O$, $V_2O_5$ et $MoO_3$, $2H_2O$.

C. Réaction de l'alcool correspondant avec l'halogénure métallique en présence d'une base anhydre. L'exemple typique est fourni par la préparation de $Th(OR)_4$ ou de $Zr(OR)_4$ :

$$ThCl_4 , 4ROH + 4NaOR \rightarrow Th(OR)_4 + 4NaCl$$
$$ZrCl_4 + 4ROH + 4NH_3 \rightarrow Zr(OR)_4 + NH_4Cl$$

On peut mettre en oeuvre cette réaction pour préparer des alcoolates de titane, d'hafnium, de germanium, de niobium, de tantale, d'aluminium et d'étain.

D. Transestérification des alcoolates métalliques d'alcools inférieurs tels que méthylates, éthylates ou isopropylates avec un alcool supérieur.

L. Brown et K. Mazdiyasni [Inorganic Chemistry, (1970) 2783] ont préconisé la méthode A pour la préparation d'un certain nombre d'alcoolates d'yttrium, de lanthane et d'autres lanthanides. Auparavant, on pensait que la réaction ne pouvait être mise en oeuvre que pour les métaux alcalins, le magnésium et l'aluminium ; L. Brown et coll. l'ont étendue à la synthèse de l'yttrium et de tous les isopropylates de lanthanides. Pour les lanthanides inférieurs tels que le lanthane, le cérium, le praséodyme et le néodyme, on utilise comme catalyseur un mélange de $HgCl_2$ et de $Hg(C_2H_3O_2)_2$ ou de $HgI_2$ pour augmenter à la fois la vitesse de réaction et son rendement. En général, on fait réagir 5 g de tournure de métal avec environ 300 cm³ d'alcool isopropylique à la température de reflux pendant environ 24 heures, en présence d'une petite quantité de catalyseur à base de sel de mercure. Les rendements seraient de 75 % ou plus.

La plupart des autres exemples que l'on trouve dans la littérature consacrée à la préparation des alcoolates de lanthanides se réfèrent à la mise en oeuvre des halogénures métalliques correspondants. Dans certains cas, on préfère utiliser à la place de $LaCl_3$, un complexe $LaCl_3$, 3ROH. [Misra et coll., Austr. J. Chem. 21 797 (1978) et Mehrotra et Batinara, Inorganic Chem 9 2505 (1970)].

Tripathi, Batwara et Mehrotra [J.C.S.A. (1967) 991] ont proposé une variante intéressante de la méthode D. Ils ont synthétisé des alcoolates d'ytterbium inférieurs (tels que le méthylate et l'éthylate) à partir d'isopropylate d'ytterbium par transestérification avec du méthanol ou de l'éthanol. En raison de leur faible solubilité, les alcools en question ont été éliminés par précipitation durant la réaction, permettant ainsi de parachever la transestérification.

En général, les méthodes A, B et C conviennent uniquement à la préparation d'alcoolates inférieurs tels que méthylates, éthylates et isopropylates étant donné que la réactivité des alcools supérieurs diminue lorsque leur poids moléculaire augmente. Il est préférable de préparer les alcoolates supérieurs à l'aide de la méthode D qui est un procédé en deux étapes.

La seule méthode connue de préparation d'alcoolates cériques appliquait la méthode C au chlorure cérique, Bradley et coll., [J.C.S. (1956) 2260-64]. Mais comme le tétrachlorure de cérium est instable, Bradley et coll. avaient choisi le complexe d'hexachlorocérate (IV) de dipyridinium comme produit de départ.

Dans un premier temps, le dioxyde de cérium est transformé en sulfate cérique d'ammonium. On précipite l'hydroxyde cérique pur à partir d'une solution aqueuse de sulfate cérique d'ammonium et on le lave à fond. On traite l'hydroxyde cérique, fraîchement préparé, en suspension dans de l'alcool absolu avec de l'acide chlorhydrique anhydre et l'on ajoute ensuite de la pyridine pour obtenir le complexe insoluble d'hexachlorocérate (IV) de dipyridinium $(Py)_2CeCl_6$. On filtre et sèche le complexe et on l'utilise pour préparer directement le méthylate, l'éthylate et l'isopropylate ; les alcoolates de propyle, butyle, sec-butyle, néopentyle et de n-pentyle étant obtenus par transalcoolyse, c'est-à-dire par transestérification, à partir de l'isopropylate. Le méthylate et l'éthylate sont aussi obtenus par transalcoolyse à partir de l'isopropylate.

L'invention selon notre demande de brevet européen 0134161 (déposée sous priorité du 9 août 1983 de la demande US Serial n° 521 787) concerne un procédé de préparation d'alcoolates cériques qui consiste à faire réagir l'hexanitratocérate (IV) d'ammonium appelé plus communément nitrate céri-ammoniacal avec un alcool, dans des conditions anhydres, en présence d'une base anhydre, à une température comprise entre environ -30°C et environ 200°C, de préférence entre environ 0°C et environ 150°C, jusqu'à formation de l'alcoolate cérique et du sel nitrate de la base.

Ce procédé évite d'avoir, dans un premier temps, à préparer, comme l'ont décrit Bradley et coll. l'hydroxyde cérique à partir du sel cérique, dans leur cas à partir du sulfate cérique d'ammonium, et à convertir ensuite l'hydroxyde en chlorure qui doit être stabilisé sous forme d'un complexe pyridinium. Le procédé de l'invention est direct, économique et, de plus, il utilise un nitrate céri-ammoniacal que l'on trouve dans le commerce et qui est relativement bon marché.

La présente invention a pour objet un procédé de préparation d'alcoolates cériques caractérisé par le fait qu'il consiste à faire réagir le nitrate céri-ammoniacal complexé avec et en solution dans un solvant choisi dans le groupe constitué par les éthers glycoliques et le tétrahydrofurane avec un alcoolate de métal alcalin, dans des conditions anhydres, à une température comprise entre environ -30°C et environ 200°C, jusqu'à formation de l'alcoolate cérique et du sel nitrate du métal alcalin.

Conformément à la présente invention, on fait appel aux éthers glycoliques. Les éthers d'alcools aliphatiques polyhydroxylés ayant de 4 à 50 atomes de carbone sont préférés. A titre d'exemples d'éthers glycoliques convenant à l'invention, on peut citer :
- l'éther diméthylique de diéthanol-1,2 ou diméthoxy-1,2 éthane (D.M.E.)
- l'éther diéthylique de diéthanol-1,2
- l'éther dibutylique de diéthanol-1,2
- l'éther diméthylique de dipropanol-1,2 ou diméthoxy-1,2 propane
- l'éther diméthylique de dipropanol-1,3 ou diméthoxy-1,3 propane (D.M.P.)
- l'éther diméthylique de diéthylèneglycol (diglyme)
- l'éther diéthylique de diéthylèneglycol
- l'éther dibutylique de diéthylèneglycol
- l'éther diméthylique de triéthylèneglycol
- l'éther diméthylique de tétraéthylèneglycol
- l'éther diméthylique de dipropylèneglycol
- l'éther diméthylique $\alpha,\gamma$ de glycérol
- l'éther diisoamylique $\alpha,\gamma$ de glycérol

A titre de solvants préférés de l'invention, on choisit le D.M.E., le D.M.P., le diglyme et le T.H.F.

Le nitrate qui se forme au cours de la réaction est insoluble dans les éthers glycoliques et le tétrahydrofuranne et peut être séparé du mélange réactionnel. L'alcoolate cérique, lorsqu'il est soluble dans le les éthers glycoliques ou dans le tétrahydrofuranne peut être séparé de la solution sous forme pure ou sous forme de complexe avec l'alcool ou le solvant. Dans certains cas, on peut employer les alcoolates en présence des nitrates sans les avoir séparés du mélange réactionnel.

Les éthers glycoliques tels que le D.M.E., le D.M.P., le diglyme et le T.H.F. forment avec le nitrate céri-ammoniacal, des complexes dont les liaisons sont tellement solides que la réaction ne se développe pas avec la base, l'ammoniac comme c'est le cas dans la demande EP 0134161. Cependant, les alcoolates de métaux alcalins, notamment des alcools aliphatiques inférieurs, qui sont définis plus loin et parmi lesquels figurent les alcoolates de lithium, de sodium et de potassium, sont réactifs.

Le D.M.E., le D.M.P., le diglyme et le T.H.F. sont aussi d'excellents solvants de l'isopropylate cérique et des alcoolates cériques ayant au moins 4 atomes de carbone, ce qui, lors du traitement du mélange réactionnel facilite la séparation de l'alcoolate cérique, puisque le sel nitrate minéral est insoluble dans lesdits solvants. Ces solvants étant solubles dans l'eau, il en résulte aussi que les solutions d'alcoolate cérique obtenues conviennent au procédé sol-gel de clivage par hydrolyse. Les produits obtenus sont utilisés dans la fabrication de céramiques spéciales.

On pense que les alcoolates cériques existent sous la forme de l'alcoolate et de complexes par association avec l'alcool libre ou avec d'autres composés utilisés comme solvants tels que par exemple le D.M.E., le D.M.P., le diglyme ou la T.H.F. Les mélanges de tous ces produits constituent ce que l'on appelle communément "alcoolate de cérium". C'est dans ce sens, communément accepté, que l'on utilise ce terme ici.

Le procédé est mis en oeuvre aisément avec les alcoolates de métal alcalin de la plupart des alcools, étant donné qu'on peut les préparer dans ces solvants, de façon relativement aisée. Il s'agit des alcoolates de métal alcalin des alcools aliphatiques inférieurs ayant de 1 à 5 atomes de carbone, tels que par exemple le méthanol, l'éthanol, le propanol, l'alcool isopropylique, le butanol, l'alcool isobutylique, l'alcool sec-butylique, l'alcool tert-butylique, le pentanol, l'alcool isopentylique, l'alcool sec-pentylique et l'alcool tert-pentylique ainsi que des alcools aliphatiques supérieurs ayant au moins 6 et jusqu'à environ 20 atomes de carbone, tels que par exemple, l'hexanol, l'heptanol, l'alcool isoheptylique, l'octanol, l'alcool isooctylique,l'éthyl-2 hexanol, l'alcool sec-octylique, l'alcool tert-octylique, le nonanol, l'alcool isononylique, le décanol, le dodécanol, le tétradécanol, l'octadécanol, l'hexadécanol, l'alcool oléylique et l'alcool eicosylique. On peut mettre en oeuvre aussi des alcools cycloaliphatiques ayant de 3 à environ 20 atomes de carbone, tel que par exemple le cyclopropanol, le cyclobutanol, le cyclopentanol, le cyclohexanol, le cycloheptanol, le cyclooctanol, le cyclododécanol, le tripropylcyclohexanol, le méthylcyclohexanol et le méthylcycloheptanol, ou bien un alcool aliphatique porteur d'un groupement aromatique ayant de 7 à environ 20 atomes de carbone tel que par exemple l'alcool benzylique, l'alcool phénéthylique, l'alcool phénylpropylique, l'alcool phényloctadécylique et l'alcool naphtyldécylique.

En ce qui concerne l'alcoolate de métal alcalin mis en oeuvre, on peut utiliser celui du commerce ou le préparer selon n'importe quelle méthode connue. On peut le préparer avant la réaction avec NaOH, en mettant en oeuvre soit un excès d'alcool, soit la quantité stoechiométrique d'alcool, en solution dans le D.M.E., le D.M.P., le diglyme ou le T.H.F. Il n'est pas nécessaire, mais il n'y a pas d'objection non plus à mettre en oeuvre un excès d'alcool libre correspondant. En conséquence, les réactions décrites plus haut peuvent être effectuées en présence d'un excès d'alcool qui est soluble dans le D.M.E., le D.M.P., le diglyme et le T.H.F. et qui est, aussi, dans la plupart des cas, un solvant de l'alcoolate cérique correspondant.

La réaction terminée on peut, après séparation du sel nitrate du métal alcalin qui est un sous-produit de la réaction, éliminer aisément le solvant par distillation sous pression atmosphérique ou réduite.

La réaction se déroule dans des conditions anhydres à une température qui peut varier dans de larges limites entre environ -30°C et environ 200°C, de préférence entre environ 0°C et environ 150°C, et encore plus préférentiellement à la température ambiante, température dépendant du système de solvant et de l'alcoolate de métal alcalin employés.

La durée de la réaction n'est pas critique. Dans le cas des alcoolates inférieurs, la réaction est presque instantanée. On poursuit la réaction jusqu'à formation de l'alcoolate désiré. La réaction peut avoir une durée de 10 minutes à plusieurs heures, mais il n'est pas nécessaire de la poursuivre au-delà de 5 heures. Habituellement, il faut de une à trois heures pour que la réaction soit terminée.

La réaction peut se dérouler très rapidement à température ambiante et, si c'est le cas, il est très vraisemblable qu'elle aura lieu à des températures bien en-dessous de la température ambiante et ce, jusqu'à -30°C, mais il n'y a aucune raison d'engager des frais supplémentaires pour refroidir le mélange réactionnel. La limite supérieure de la température réactionnelle est imposée par la volatilité du mélange réactionnel ou de l'un de ses composants, et sa température de décomposition. Il n'y a aucune raison de mettre en oeuvre une température supérieure au point d'ébullition du mélange réactionnel à la pression atmosphérique, mais si la température d'ébullition est trop basse, on peut utiliser un réacteur fermé ou un appareillage sous pression (par exemple de 2 à 3 atmosphères : $2{,}026 \cdot 10^5$ à $3{,}039 \cdot 10^5$ Pa). Les facteurs ci-dessus étant pris en considération, il est inutile que la température réactionnelle dépasse 200°C.

La quantité de base anhydre à savoir l'alcoolate de métal alcalin est stoechiométrique étant donné que la fonction du cation de la base, le métal alcalin est de fixer le nitrate provenant du nitrate ceri-ammoniacal de départ :

$$Ce(NO_3)_6\ (NH_4)_2\ +\ 6\ Na\ OR \rightarrow 6\ Ne\ NO_3\ +\ 2\ NH_3\ +\ Ce\ (OR)_4\ +\ 2\ ROH$$

Il est possible, mais non nécessaire, de mettre en oeuvre un excès qui, dans certains cas où il est difficile à séparer de l'alcoolate cérique, doit être évité.

L'équation montre qu'il n'y a pas besoin d'alcool additionnel. Cependant, il est possible de mettre en oeuvre un excès d'alcool libre.

Le mélange réactionnel contient le sel nitrate de métal alcalin qui peut être séparé de l'alcoolate cérique au cours du procédé. Si ce sel est moins soluble dans le mélange réactionnel que l'alcoolate obtenu, on peut le séparer du produit réactionnel par filtration. Il existe une autre possibilité qui consiste à reprendre le mélange réactionnel dans un solvant inerte tel que benzène, toluène ou hexane, de préférence dans un solvant inerte dans lequel l'alcoolate obtenu est soluble et le sel nitrate insoluble, le sel nitrate étant séparé ensuite par filtration ou centrifugation.

Dans le cas des alcools inférieurs en-dessous de l'isopropylate, l'alcoolate cérique obtenu ainsi que le

4

sel nitrate de métal alcalin sont solides et insolubles tous les deux dans le D.M.E., le D.M.P., le diglyme et le T.H.F. Dans ce cas, naturellement, il n'est pas possible de les séparer par filtration ; on peut récupérer l'alcoolate et le séparer du sel nitrate par extraction avec un solvant de l'alcoolate dans lequel le sel nitrate est insoluble, en utilisant, par exemple, un appareil Soxhlet. Une autre possibilité consiste à utiliser un solvant du sel nitrate de métal alcalin dans lequel l'alcoolate cérique est insoluble. Par exemple, le nitrate de sodium est soluble dans le méthanol alors que le tétraméthylate cérique ne l'est pas et, par conséquent, on peut, dans ce cas, séparer le nitrate de sodium par extraction avec le méthanol.

En fonction des conditions de réaction et de mise en oeuvre, on peut isoler l'alcoolate sous forme d'associations avec une ou plusieurs molécules d'alcool qui, généralement, le rendent plus stable à l'hydrolyse. Par ailleurs, on peut isoler l'alcoolate sous une forme partiellement hydrolysée convenant ou appropriée à certaines applications.

Pour certaines applications on peut utiliser les alcoolates de cérium sous la forme sous laquelle ils existent dans le mélange réactionnel à la fin de la réaction sans avoir à les isoler du mélange réactionnel ou à les séparer des nitrates ce qui évite des coûts de traitement et de manipulation.

Les exemples qui suivent illustrent un mode de réalisation préférentielle de l'invention, mais, en aucun cas, ils ne limitent la portée de l'invention.

EXEMPLE 1 -

Préparation de tétraisopropylate cérique à partir de nitrate céri-ammoniacal et d'isopropylate de sodium en solution dans du diméthoxy-1,2 éthane et de l'alcool isopropylique.

a) Réactifs :

| Réactifs | Poids(g) | Teneur (%) | Poids molaire | Moles | Rapport molaire | Remarques |
|---|---|---|---|---|---|---|
| Sodium | 4,14 | - | 22,99 | 0,18 | 6,00 | |
| Alcool isopropylique | 55,71 | - | 60,09 | 0,93 | 30,90 | |
| Nitrate cériammoniacal | 16,45 | OT*=31,15 | 548,26 | 0,03 | 1,00 | séché à l'étuve 115°C |
| Diméthoxy-1,2 éthane | 66,15 | - | 90,12 | 0,73 | 24,47 | séché et distillé sur Li Al H$_4$ |

* pourcentage exprimé en oxyde de terre rare (le sodium et l'alcool isopropylique donnent une solution à 24,73 % d'isopropylate de sodium dans l'alcool isopropylique).

b) Mode opératoire :

On sèche l'appareillage dans une étuve à 115°C. On conduit toutes les réactions sous atmosphère d'argon. On charge l'alcool isopropylique dans un ballon de 250 cm$^3$ à fond rond placé dans une boîte à gants, puis on le retire et on l'équipe d'un agitateur magnétique et d'un tube de Claisan qui, à son tour, est muni d'un thermomètre et d'un réfrigérant. Le réfrigérant est équipé d'un cube en T relié à un barboteur et à une source d'argon. Avant d'ouvrir le ballon pour y ajouter la quantité requise de sodium, on augmente le débit d'argon. On chauffe le mélange sous reflux pendant environ 3 heures pour achever la réaction. Ensuite, on remet le ballon à fond rond dans la boîte à gants (toujours maintenu sous argon) et on l'équipe d'une ampoule de coulée, chargée de la solution orange de nitrate céri-ammoniacal dans du diméthoxy-1,2 éthane. On maintient la température au-dessus de 75°C. On ajoute lentement, goutte à goutte, la solution de nitrate céri-ammoniacal. Il se produit une légère réaction exothermique lorsque la solution vire progressivement au jaune et devient plus fluide. On peut voir un précipité se former dans le ballon. Lorsque l'addition est terminée, on agite le mélange pendant une heure et on filtre sur un verre fritté et on lave quatre fois au diméthoxy-1,2 éthane. On sèche ensuite le solide blanc de NaNO$_3$ et l'on sépare l'alcool isopropylique et le diméthoxy-1,2 éthane du filtrat par distillation. On obtient alors un solide jaune brillant qui est le tétraisopropylate cérique.

c) Résultats :

On obtient 15,2 g de NaNO$_3$ (théorie : 15,3 g), solide blanc contenant une très faible quantité de cérium. Le rendement en nitrate de sodium est de 99,3 %. Le tétraisopropylate cérique est isolé sous la forme d'un solide jaune (Trouvé : 10,3 g, théorie : 11,29 g). Il se produit quelques pertes au cours du procédé. Cendres du produit trouvé : 49,6 %, théorie : 45,7 %.

EXEMPLE 2 -

Préparation de tétraisopropylate cérique à partir de nitrate céri-ammoniacal, d'isopropylate de sodium en solution dans du diméthoxy-1,2 éthane , sans alcool isopropylique libre

a) Réactifs :

| Réactifs | Poids(g) | Teneur (%) | Poids molaire | Moles | Rapport molaire aire | Remarques |
|----------|----------|------------|---------------|-------|----------------------|-----------|
| Sodium | 4,83 | - | 22,99 | 0,21 | 6,00 | |
| Alcool isopropylique | 12,62 | - | 60,09 | 0,21 | 6,00 | |
| Diméthoxy-1,2 éthane | 50,60 | - | 90,12 | 0,56 | 16,04 | pour l'isopropylate de sodium |
| Diméthoxy-1,2 éthane | 29,40 | - | 90,12 | 0,326 | 9,32 | pour la solution de nitrate cériammoniacal |
| Nitrate cériammoniacal | 19,20 | OT = 31,15 | 548,26 | 0,035 | 1,00 | séché à l'étuve 115°C |

b) Mode opératoire :

On introduit 12,62 g d'alcool isopropylique et 50,6 g de diméthoxy-1,2 éthane dans un ballon à fond rond de 250 cm$^3$ placé à l'intérieur d'une boîte à gants, on le retire et on l'équipe d'un agitateur magnétique et d'un tube de Claisen qui, à son tour, est muni d'un thermomètre et d'un réfrigérant. Le réfrigérant est équipé d'un tube en T relié à un barboteur et à une source d'argon. Avant d'ouvrir le ballon pour ajouter la quantité requise de sodium, on augmente le débit d'argon. Le mélange est chauffé sous reflux à 90°C pendant environ 24 heures pour achever la réaction. On remet le ballon à fond rond dans la boîte à gants (toujours maintenue sous argon) et on l'équipe d'une ampoule de coulée chargée de la solution de nitrate céri-ammoniacal dans 29,4 g de diméthoxy-1,2 éthane. La température est maintenue au-dessus de 75°C pendant que l'on ajoute goutte à goutte la solution de nitrate céri-ammoniacal. Il se produit une réaction légèrement exothermique lorsque la solution vire progressivement au jeune et devient plus fluide. On peut voir un précipité se former dans le ballon. Lorsque l'addition est terminée, on agite le mélange pendant une heure, on le filtre ensuite sur un verre fritté et on le lave quatre fois avec du diméthoxy-1,2 éthane. On sèche ensuite le solide blanc de NaNO$_3$ et l'on sépare le diméthoxy-1,2 éthane du filtrat par distillation. On obtient un solide jaune brillant qui est le tétraisopropylste cérique.

Les résultats sont analogues à ceux obtenus dans l'exemple 1.

EXEMPLE 3 -

Préparation de tétraisopropylate cérique à partir de nitrate céri-ammoniacal et d'isopropylate de sodium en solution dans du tétrahydrofuranne et de l'alcool isopropylique

| Réactifs | Poids(g) | Teneur (%) | Poids molaire | Moles | Rapport molaire | Remarques |
|----------|----------|------------|---------------|-------|-----------------|-----------|
| Sodium | 4,14 | - | 22,99 | 0,18 | 6,00 | |
| Alcool isopropylique | 59,81 | - | 60,09 | 0,99 | 33,20 | |
| Nitrate cériammoniacal | 16,45 | OT = 31,15 | 548,26 | 0,03 | 1,00 | séché à l'étuve 115°C |
| Tétrahydrofuranne | 72,25 | - | 72,11 | 1,00 | 33,40 | séché et distillé sur Li Al H$_4$ |

b) <u>Mode opératoire :</u>

On charge l'alcool isopropylique dans un ballon à fond rond de 250 cm$^3$ placé à l'intérieur d'une boîte à gants, on le retire et on l'équipe d'un agitateur magnétique et d'un tube de Claisen qui est muni à son tour d'un thermomètre et d'un réfrigérant. Le réfrigérant est équipé d'un tube en T relié à un barboteur et une source d'argon. Avant d'ouvrir le ballon pour ajouter le quantité requise de sodium, on augmente le débit d'argon. Le mélange est chauffé sous reflux pendant environ 3 heures pour achever la réaction. On replace le ballon à fond rond dans la boîte à gants (toujours maintenue sous argon). A l'aide d'une ampoule de coulée, on ajoute lentement, goutte à goutte, la solution orange de nitrate céri-ammoniacal dans le tétrahydrofuranne. La température est maintenue au-dessus de 75°C. Il se produit une légère réaction exothermique lorsque la solution vire progressivement au jaune et devient plus fluide. On observe un précipité dans le ballon. Lorsque l'addition est terminée, on agite le mélange pendant une heure, ensuite on le filtre sur un verre fritté et on le lave quatre fois au tétrahydrofuranne. On sèche ensuite le solide blanc de NaNO$_3$ et l'on sépare l'alcool isopropylique et le tétrahydrofuranne du filtrat par distillation. On isole un solide jaune brillant qui est le tétraisopropylate cérique.

Les résultats sont analogues à ceux de l'exemple 1.

<u>EXEMPLE 4 -</u>

<u>Préparation de tetraméthylate cérique à partir de nitrate céri-ammoniacal et de méthylate de sodium en solution dans du diméthoxy-1,2 éthane</u>

a) <u>Réactifs :</u>

| Réactifs | Poids(g) | Teneur (%) | Poids molaire | Moles | Rapport molaire | Remarques |
|---|---|---|---|---|---|---|
| Solution de méthylate de sodium | 37,98 | 25,6 % p/p | 54,02 | 0,180 | 6,00 | Solution MeOH du commerce |
| Nitrate cériammoniacal | 16,45 | OT = 31,15 | 548,26 | 0,030 | 1,00 | séché à l'étuve 115°C |
| Diméthoxy-1,2 éthane | 110,9 | - | 90,12 | 1,23 | 41,0 | séché et distillé sur Li Al H$_4$ |

b) <u>Mode opératoire :</u>

On introduit le nitrate céri-ammoniacal et le diméthoxy-1,2 éthane dans un ballon à fond rond de 250 cm$^3$ muni d'un agitateur et placé dans une boîte à gants emplie d'argon. Après la dissolution du nitrate céri-ammoniacal, on ajoute goutte à goutte la solution de méthylate de sodium en 15 minutes au moyen d'une ampoule munie d'un tube latéral de réduction de la pression. On agite la bouillie jeune brillant pendant une heure. On sépare les solides par filtration, on les introduit immédiatement dans un appareil Soxhlet, préalablement séché, et on procède à l'extraction eu MeOH durant la nuit.

On évapore à sec et l'on obtient 15,2 g de nitrate de sodium (théorie : 15,3 g). Après séchage, la cartouche contient 7,9 g de tétraméthylate cérique produit jaune brillant (théorie : 7,93 g).

<u>EXEMPLE 5 -</u>

<u>Préparation de tetrabutylate cérique à partir de nitrate céri-ammoniacal et de butylate de sodium en solution dans du diméthoxy-1,2 éthane et de l'alcool n-butylique</u>

a) <u>Réactifs :</u>

| Réactifs | Poids(g) | Teneur (%) | Poids molaire | Moles | Rapport molaire | Remarques |
|---|---|---|---|---|---|---|
| Sodium | 4,14 | - | 22,99 | 0,18 | 6,00 | solide |
| Alcool n-butylique | 52,36 | - | 74,12 | 0,71 | 23,7 | séché avec sodium et distillé |
| Nitrate cériammoniacal | 16,45 | OT = 31,15 | 548,26 | 0,03 | 1,00 | séché à l'étuve 115°C |
| Diméthoxy-1,2 éthane | 51,45 | - | 90,12 | 0,57 | 19,0 | séché et distillé sur Li Al $H_4$ |

b) <u>Mode opératoire</u>

On introduit le n-butanol dans un ballon à fond rond de 250 cm$^3$ placé dans une boîte à gants, on le retire et on l'équipe d'un agitateur magnétique et d'un tube de Claisen muni, à son tour, d'un thermomètre et d'un réfrigérant. Le réfrigérant est équipé d'un tube en T relié à un barboteur et à une source d'argon. Avant d'ouvrir le ballon pour ajouter la quantité requise de sodium, on augmente le débit d'argon. On chauffe le mélange eu reflux à 118°C afin d'achever la réaction. Ensuite, on remet le ballon à fond rond dans la boîte à gants (toujours maintenue sous argon). On ajoute goutte à goutte la solution orange de nitrate céri-ammoniacal dans le diméthoxy-1,2 éthane au moyen d'une ampoule de coulée. La température est maintenue au-dessus de 75°C tandis que l'on ajoute lentement, goutte à goutte, la solution de nitrate céri-ammoniacal. Il se produit une légère réaction exothermique lorsque la solution vire progressivement au jaune et devient plus fluide. On peut observer un précipité dans le ballon. L'addition terminée, on agite pendant une heure. On sépare le $NaNO_3$ par filtration et on le lave quatre fois avec du diméthoxy-1,2 éthane (trouvé : 15,3 g, théorie : 15,3 g). On concentre le filtrat jaune sous pression réduite pour obtenir 12,8 g de tétrabutylate cérique sous forme de solide jaune brillant (théorie : 12,9 g).

**Revendications**

1. Procédé de préparation d'alcoolates cériques caractérisé par le fait qu'il consiste à faire réagir le nitrate céri-ammoniacal complexé avec et en solution dans un solvant choisi dans le groupe constitué par les éthers glycoliques et le tétrahydrofuranne, avec un alcoolate de métal alcalin en présence éventuellement de l'alcool correspondant à l'alcoolate de métal alcalin, dans des conditions anhydres, à une température comprise entre environ -30°C et environ 200°C, jusqu'à formation de l'alcoolate cérique et du sel nitrate du métal alcalin.

2. Procédé selon la revendication 1 caractérisé par le fait que l'alcool correspondant à l'alcoolate du métal alcalin est présent dans le mélange réactionnel.

3. Procédé selon la revendication 1 caractérisé par le fait que l'alcoolate de métal alcalin dérive d'un alcool aliphatique ayant de un jusqu'à environ 20 atomes de carbone.

4. Procédé selon la revendication 1 caractérisé en ce que l'alcoolate de métal alcalin dérive d'un alcool cycloaliphatique ayant de 3 à environ 20 atomes de carbone.

5. Procédé selon la revendication 1 caractérisé en ce que l'alcoolate de métal alcalin dérive d'un alcool aliphatique porteur d'un groupement aromatique ayant de 7 à environ 20 atomes de carbone.

6. Procédé selon la revendication 1 caractérisé en ce que le solvant est le diméthoxy-1,2 éthane.

7. Procédé selon la revendication 1 caractérisé en ce que le solvant est le diméthoxy-1,3 propane.

8. Procédé selon la revendication 1 caractérisé en ce que le solvant est le tétrahydrofuranne.

9. Procédé selon la revendication 1 caractérisé en ce que le solvant est le diméthoxy -1, 2 propane.

**Claims**

1. Process for the preparation of ceric alcoholates, characterised in that it consists in reacting ammonium ceric nitrate complexed with and in solution in a solvent chosen from the group comprising glycol ethers and tetrahydrofuran, with an alkali metal alcoholate, optionally in the presence of the alcohol corresponding to the alkali metal alcoholate, under anhydrous conditions, at a temperature of between about -30°C and about 200°C, until the ceric alcoholate and the alkali metal nitrate salt are formed.

2. Process according to Claim 1, characterised in that the alcohol corresponding to the alkali metal alcoholate is present in the reaction mixture.

3. Process according to Claim 1, characterised in that the alkali metal alcoholate is derived from an aliphatic alcohol containing from one up to about 20 carbon atoms.

4. Process according to Claim 1, characterised in that the alkali metal alcoholate is derived from a cycloaliphatic alcohol containing from 3 to about 20 carbon atoms.

5. Process according to Claim 1, characterised in that the alkali metal alcoholate is derived from an aliphatic alcohol carrying an aromatic group containing from 7 to about 20 carbon atoms.

6. Process according to Claim 1, characterised in that the solvent is 1,2-dimethoxyethane.

7. Process according to Claim 1, characterised in that the solvent is 1,3-dimethoxypropane.

8. Process according to Claim 1, characterised in that the solvent is tetrahydrofuran.

9. Process according to Claim 1, characterised in that the solvent is 1,2-dimethoxypropane.

**Patentansprüche**

1. Verfahren zur Herstellung von Ceralkoholaten, dadurch gekennzeichnet, daß als Komplex mit und als Lösung in einem aus der Gruppe von Glykolethern und Tetrahydrofuran ausgewähltem Lösungsmittel vorliegendes Ammonium-hexanitratocerat (IV) mit dem Alkoholat eines Alkalimetalls, gegebenenfalls in Gegenwart des dem Alkalialkoholat entsprechenden Alkohols unter wasserfreien Bedingungen und bei einer Temperatur zwischen etwa -30°C und etwa 200°C bis zur Bildung von Ceralkoholat und Alkalinitratsalz umgesetzt wird.

2. Verfahren nach Anspruch 1, dadurch gekennzeichnet, daß der dem Alkalialkoholat entsprechende Alkohol im Reaktionsgemisch vorhanden ist.

3. Verfahren nach Anspruch 1, dadurch gekennzeichnet, daß das Alkalialkoholat von einem aliphatischen Alkohol mit bis zu etwa 20 Kohlenstoffatomen abstammt.

4. Verfahren nach Anspruch 1, dadurch gekennzeichnet, daß das Alkalialkoholat von einem cycloaliphatischen Alkohol mit 3 bis etwa 20 Kohlenstoffatomen abstammt.

5. Verfahren nach Anspruch 1, dadurch gekennzeichnet, daß das Alkalialkoholat von einem aliphatischen Alkohol abstammt, der eine aromatische Gruppe mit 7 bis etwa 20 Kohlenstoffatomen trägt.

6. Verfahren nach Anspruch 1, dadurch gekennzeichnet, daß das Lösungsmittel 1,2-Dimethoxyethan ist.

7. Verfahren nach Anspruch 1, dadurch gekennzeichnet, daß das Lösungsmittel 1,3-Dimethoxypropan ist.

8. Verfahren nach Anspruch 1, dadurch gekennzeichnet, daß das Lösungsmittel Tetrahydrofuran ist.

9. Verfahren nach Anspruch 1, dadurch gekennzeichnet, daß das Lösungsmittel 1,2-Dimethoxypropan ist.